# EUROPEAN PATENT SPECIFICATION

(11) **EP 2 908 828 B1**
(45) Date of publication and mention of the grant of the patent: **06.09.2017**
(21) Application number: 13774088.2
(22) Date of filing: 19.09.2013
(51) Int. Cl.: A61K 31/685, A61K 45/06, A61K 33/00, A61P 15/14, A61K 36/185, A61K 36/23, A61K 36/28, A61K 36/38, A61K 36/48, A61K 31/357

(54) **GALACTAGOGUE COMPOSITIONS BASED ON PHOSPHATIDYLSERINE**
GALAKTAGOGUM-ZUSAMMENSETZUNGEN AUF PHOSPHATIDYLSERIN-BASIS
COMPOSITIONS GALACTAGOGUES À BASE DE PHOSPHATIDYLSÉRINE

(30) Priority: 22.10.2012 IT MI20121784
(43) Date of publication of application: 26.08.2015
(73) Proprietor: Velleja Research SRL, 29010 Pontenure (PC) (IT)
(72) Inventor: DI PIERRO, Francesco, I-29010 Pontenure (PC) (IT)
(74) Representative: Bianchetti Bracco Minoja S.r.l.
(86) International application number: PCT/EP2013/069466
(87) International publication number: WO 2014/063868

(56) References cited:
- EP-A1- 0 148 045
- EP-A1- 0 965 597
- EP-A1- 1 774 963
- CN-A- 101 129 763
- CN-A- 102 578 436
- US-A1- 2007 160 659
- US-A1- 2007 253 941
- US-A1- 2009 110 674
- US-A1- 2012 189 734

## Description

The present invention relates to galactagogue compositions based on phosphatidylserine.

### PRIOR ART

Breast milk is a complete food for infants in both qualitative and energy-giving terms. The quantity of milk secreted daily varies at different periods, and there is a certain individual variability between women: on average, from 200 g on the 3rd day after the birth, gradually increasing to 1200-1500 ml towards the 6th month of lactation, after which it gradually declines.

The period of lactation in women corresponds to the functional activity of the breast: it consequently begins shortly before the birth, and ends at weaning. Without considering the initial period (the colostrum-producing stage), the milk-producing stage begins about 48 hours after the birth. This delay is due to the pituitary gland which, in the first few days after the birth, is still under the inhibiting influence of the placental hormones accumulated during pregnancy, and is therefore unable to produce prolactin. Lactogenesis does not take place until the 3rd/4th day after the birth.

Complete lack of milk secretion (agalactia) is exceptional. Sometimes, however, especially in primiparas, particularly low secretion may be observed in the first week after the birth (hypogalactia). Women who eliminate less than 280 g of milk in a 24-hour period on the 5th day after the birth are classed as hypogalactic (primary hypogalactia). Secondary hypogalactia appears after a period of normal milk secretion.

Primary hypogalactia, which was very rare before the 1950s, has become progressively more frequent recently. Hypogalactia can be caused by a secretion defect due to delay in lactogenesis or to particular biological, neurohumoral or psychological conditions, especially in women who go out to work, by local or general disorders, or by stagnation of the secretion (caused by insufficient suction or an obstacle to emptying).

In hypogalactia, a reduction in the blood lactose level is observed: in women with normal milk secretion the blood lactose level ranges between 0.30 and 0.80 g per thousand, whereas in women suffering from hypogalactia it is below 0.20 g per thousand. The treatment of hypogalactia is based mainly on identification and removal of the causal factor.

Some of the medicaments currently proposed for the treatment of hypogalactia, such as metoclopramide, sulpiride, chlorpromazine, domperidone and GH induce side effects (extrapyramidal and neuroleptic effects, weight and blood pressure increases) which limit their efficacy. The use of plants or extracts thereof known from traditional medicine (fenugreek, stinking tutsan, mallow, aniseed, milk thistle and goat's rue) is substantially devoid of side effects, but is still not well characterised from the pharmacological standpoint.

Phosphatidylserine (PS) belongs to the class of phospholipids, ie. molecules that constitute the cell membranes. In addition to phosphatidylserine, which is present to a low extent in animal tissues, phospholipids include phosphatidic acid, phosphatidylcholine, phosphatidylethanolamine, phosphatidylglycerophosphate, diphosphatidylglycerols and phosphatidylinositol.

It has long been known that PS has favourable effects on the memory and cognitive faculties (EP 148045). A number of studies confirm the properties of phosphatidylserine in terms of reinstatement of the memory capacity, especially if associated with aging. PS is also used as an ergogenic aid in sport, as an antidepressant in neurology, and as a sedative in anxiety.

EP 965597 discloses that the activity of a number of drugs may be improved by combining them with substances having affinity for phospholipids, particularly for phosphatidylserine.

The galactogenic activity of silymarin is disclosed in EP 1774963. US 2012/189734 discloses nutritional compositions useful for lactating women comprising proteins, lipids, carbohydrates, vitamins and herbal extracts.

US 2007/160659 and US 2007/253941 disclose pharmaceutical compositions comprising phosphatidylserine for use as nutritional supplements.

US 2009/110674 discloses compositions including inter alia phosphatidylserine and silymarin useful as anti-aging, anti-oxidant health supplement.

CN101129763 discloses a composition for treating post partum hypogalactia comprising traditional Chinese herbal extracts.

CN102578436 discloses a compositions of herbal extracts for use as feed additive for sows in perinatal period promoting milk secretion and enabling healthy piglets growth.

### DESCRIPTION OF THE INVENTION

It has now been found that phosphatidylserine (PS) performs a strong galactagogue action, increasing milk production without altering the quality of the milk. The subject of the invention is therefore compositions containing phosphatidylserine for use in the treatment of hypogalactia.

The compositions according to the invention contain the complex of silymarin with phosphatidylserine with one or more ingredients selected from goat's rue, mallow, stinking tutsan, fenugreek and aniseed, preferably silymarin and/or goat's rue. Phosphatidylserine can also be used in the form of complexes thereof with the constituents of extracts of said medicinal plants. The preparation of said extracts is known, and is disclosed, for example, in EP 209038. PS is typically administered at daily doses of between 20 and 500 mg, preferably between 20 and 250 mg, and more preferably between 20 and 100 mg, optionally divided into two or three administrations a day. The doses of the other ingredients, if any, can also vary within wide limits, corresponding to the doses already used for the same ingredients for different indications. For example, the dose of silymarin can range between 50 and 500 mg, in particular between 100 and 200 mg.

The efficacy of PS was evaluated on the female Wistar rat (weight 300 g ± 35): the administration of 100 mg/kg/os/day produced an increase in the plasma prolactin value over 25% higher than that obtained by administering metoclopramide at the dose of 2.2 mg/kg/ip/day. The administration of PS, again at the dose of 100 mg/kg/os/day, also antagonised the anti-galactagogue effect obtained by administering 1 mg/kg/ip/day of bromocriptine.

The galactagogue action of PS was also confirmed in a clinical trial conducted on 2 groups of 10 women, who for ethical reasons had already reached the 4th month of lactation. The first group was treated with 30 mg/day of PS for 30 consecutive days, and the second with a placebo.

It was observed at the end of the trial that the quantity of milk produced by the women in the group treated with PS was about 30% higher than the same figure (at t=30) for the placebo group (which increased by about 15% compared with t=0). Said modifications were solely quantitative: the biochemical parameters (water, fats, carbohydrates and proteins) remained unchanged in both groups.

The compositions according to the invention can be formulated in a way suitable for oral administration, and will be prepared by conventional methods well known in pharmaceutical technology, such as those described in "Remington's Pharmaceutical Handbook", Mack Publishing Co., N.Y., USA, using excipients, diluents, fillers, anti-caking agents, flavourings and sweeteners acceptable for their final use. Examples of suitable formulations include capsules, tablets, granulates, powders, solutions and suspensions. PS can optionally be introduced into suitable functional foods.

An example of a formulation in single-dose sachets is set out below:

| | |
|---|---|
| Phosphatidylserine | 28 mg |
| Silymarin | 140 mg |
| Goat's rue | 200 mg |
| Refined sucrose | 4000 mg |
| Anhydrous citric acid | 200 mg |
| Sucrose ester | 30 mg |
| Orange flavouring | 200 mg |

## Claims

1. Phosphatidylserine for use in the treatment of hypogalactia.

2. Compositions comprising phospatidylserine complexed with silymarin and one or more ingredients selected from goat's rue, mallow, stinking tutsan, fenugreek and aniseed.

3. Compositions according to claim 2, comprising goat's rue.

## Patentansprüche

1. Phosphatidylserin zur Verwendung bei der Behandlung von Hypogalaktie.

2. Zusammensetzungen, enthaltend Phosphatidylserin, komplexiert mit Silymarin und einem oder mehreren Inhaltsstoffen, ausgewählt aus Geißraute, Malve, Bocks-Johanniskraut, Bockshornklee und Anis.

3. Zusammensetzungen gemäß Anspruch 2, enthaltend Geißraute.

## Revendications

1. Phosphatidylsérine pour l'utilisation dans le traitement de l'hypolactation.

2. Compositions comprenant de la phosphatidylsérine complexée avec de la silymarine et un ou plusieurs ingrédients sélectionnés parmi la rue des chèvres, la mauve, le millepertuis à odeur de bouc, le fenugrec et la graine d'anis.

3. Compositions selon la revendication 2, comprenant de la rue des chèvres.
